(19) Europäisches Patentamt
European Patent Office
Office européen des brevets

(11) **EP 2 319 964 B1**

(12) **EUROPEAN PATENT SPECIFICATION**

(45) Date of publication and mention
of the grant of the patent:
**20.05.2015 Bulletin 2015/21**

(21) Application number: **09810069.6**

(22) Date of filing: **25.08.2009**

(51) Int Cl.:
*D01F 1/10* *(2006.01)*       *D01F 8/14* *(2006.01)*
*D01D 5/36* *(2006.01)*       *D01F 6/62* *(2006.01)*
*B01J 35/02* *(2006.01)*       *B01J 35/06* *(2006.01)*
*D01F 6/92* *(2006.01)*       *D01F 8/04* *(2006.01)*

(86) International application number:
**PCT/JP2009/065137**

(87) International publication number:
**WO 2010/024423 (04.03.2010 Gazette 2010/09)**

(54) **MANUFACTURING METHOD FOR ULTRAFINE FIBERS CONTAINING DEODORIZING AGENT**

HERSTELLUNGSVERFAHREN FÜR ULTRAFEINE FASERN MIT EINEM DESODORIERENDEN WIRKSTOFF

PROCÉDÉ DE FABRICATION DE FIBRES ULTRAFINES CONTENANT UN AGENT DÉSODORISANT

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **27.08.2008   JP 2008218079**

(43) Date of publication of application:
**11.05.2011   Bulletin 2011/19**

(73) Proprietor: **TEIJIN LIMITED**
**Chuo-ku**
**Osaka-shi**
**Osaka 541-0054 (JP)**

(72) Inventors:
• **OHTA, Masami**
**Matsuyama-shi**
**Ehime 791-8041 (JP)**
• **MORISHIMA, Kazuhiro**
**Matsuyama-shi**
**Ehime 791-8041 (JP)**

(74) Representative: **Hallybone, Huw George**
**Carpmaels & Ransford LLP**
**One Southampton Row**
**London WC1B 5HA (GB)**

(56) References cited:
WO-A1-2007/078203       WO-A2-2008/000198
JP-A- 8 284 011       JP-A- 2001 355 119
JP-A- 2006 265 773       JP-A- 2007 015 202
JP-A- 2007 063 714       JP-A- 2009 150 024
US-A1- 2005 136 100

EP 2 319 964 B1

**Description**

Technical Field

The present invention relates to a deodorant functional agent-containing extra fine filament yarn having an excellent deodorizing function and a method for producing the same. The invention relates more specifically to an extra fine multifilament, which can hold a deodorant functional agent without dropping and can show a deodorizing function more effectively than in the past, and a method for producing the same.

Background Art

Recently, with the diversification of living environments intended for comfortable lifestyles, there has been increasing attention to various odors not only in homes but also in offices, hospitals, etc. Furthermore, with the increase in house airtightness, there has been a significant problem of treating bad odors and harmful components (such as formaldehyde) in house.

Under such circumstances, various attempts have been made to use a filament structure having a deodorizing function for removing a bad odor. A proposed filament structure has not only an adsorbent function but also a decomposition function such as a photocatalytic activity, and thereby can exhibit a permanent deodorizing function.

For example, in a conventional method, a filament structure is subjected to an aftertreatment for attaching a deodorant component, to obtain a photocatalytic deodorizing function (JP-A-2001-254281, etc.) However, in this method, deodorant functional agent particles are placed on the filament surfaces, so that the particles are easily dropped disadvantageously. Furthermore, a binder is used in the attaching treatment, so that the filament cloth per se has a hard texture disadvantageously.

In view of solving the durability and texture disadvantages, various filament structures obtained by kneading a photocatalyst into fibers have been proposed (JP-A-2005-220471, etc.) However, in this method, since the photocatalyst is embedded in the fibers, a reaction between the photocatalyst and an odorous component is inhibited by the fiber polymer, and the photocatalyst cannot sufficiently exhibit its ability. Furthermore, the matrix is disadvantageously deteriorated by the photocatalyst per se to lower the fiber strength with time. A core-sheath composite filament structure is proposed in JP-A-2004-169217, etc. in view of this disadvantage. In this structure, a photocatalyst is disposed only on the sheath portion, and the core portion ensures the strength.

However, in this method, though the strength disadvantage is solved, the photocatalyst is embedded in the sheath portion and thereby cannot sufficiently exhibit its ability. In a method proposed to solve the problems, a photocatalyst is kneaded into a peel-off dividable composite filament structure, and the structure is divided to increase the photocatalytic area exposed on the filament surfaces, so that the photocatalyst can easily exhibit its ability (JP-A-10-204727). In this method, the exposed area can be increased and the fineness can be improved, whereby the photocatalytic effect and the texture can be improved to some extent. However, this method is disadvantageous in that the structure cannot be produced with stable quality because of division ratio variation.

US2005/136100, WO2007/078203, WO2008/000198 and JP2001 355119 form part of the technological background.

Disclosure of the Invention

An object of the present invention is to solve the problems of conventional technologies, thereby providing a deodorant functional agent-containing extra fine multifilament, which exhibits an excellent deodorizing function with only a small deterioration of the initial function in long-term use, repeated washings, etc., and a method for producing the same.

As a result of intense research in view of solving the above problems, the inventors have found that the above extra fine multifilament can be obtained by controlling the diameter of fibers and the particle diameter of a deodorant functional agent contained in the fibers within particular ranges.

Thus, according to the invention, there is provided a method for producing a deodorant functional agent-containing extra fine multifilament, comprising removing a sea component from a sea-island composite filament yarn including the sea component and island component to obtain an extra fine multifilament containing an island component, characterized in that

a) the island component comprises extra fine single yarn fibers having an average diameter of 200 to 2000 nm,

b) the island component comprises at least a deodorant functional agent having a secondary particle diameter equal to or more than the diameter of the extra fine single yarn fibers, and

c) the sea-island composite filament yarn is prepared by melt spinning and direct drawing without temporal winding.

The method of the invention can provide a deodorant functional agent-containing extra fine multifilament, characterized in that

a) the multifilament comprises extra fine single yarn fibers having an average diameter of 200 to 2000 nm, and
b) the multifilament comprises at least a deodorant functional agent having a secondary particle diameter equal to or more than the diameter of the extra fine single yarn fibers, wherein 5 or more particles of the deodorant functional agent per 25 $\mu m^2$ are not completely covered with a fiber polymer and are partially exposed on the surface of the multifilament.

Brief Description of the Drawings

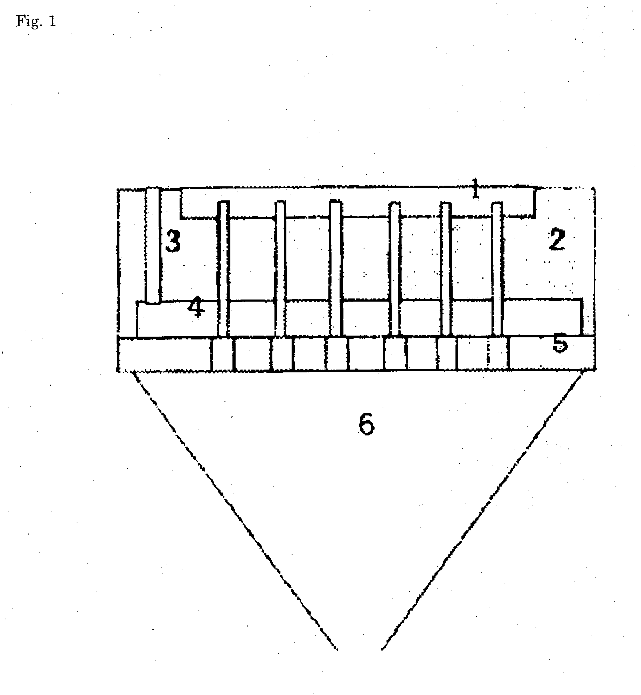

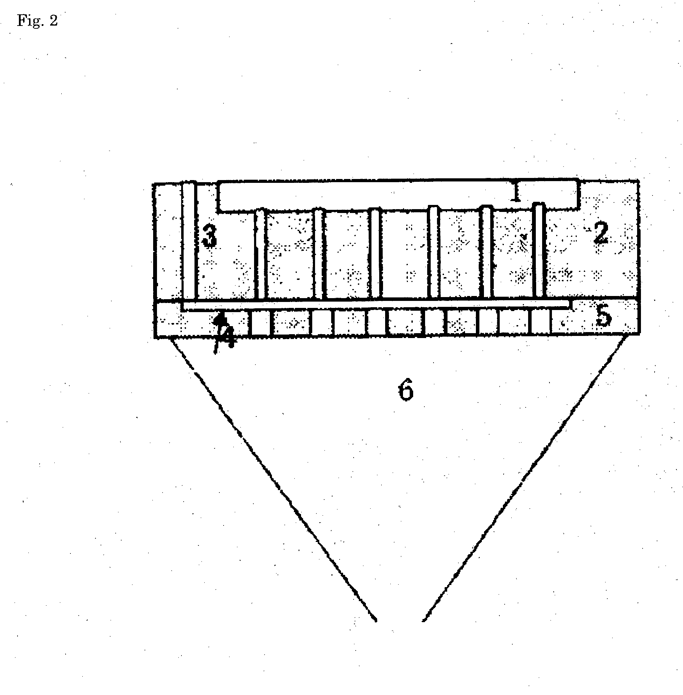

Figs. 1 and 2 are schematic, cross-sectional conceptual view each showing an example of a spinneret for preparing a sea-island composite filament yarn used in the present invention. In Figs. 1 and 2, 1 represents an undistributed island component polymer reservoir, 2 represents an island component distributing pipe, 3 represents a sea component inlet, 4 represents an undistributed sea component polymer reservoir, 5 represents an individual sea/island structure forming portion, and 6 represents an entire combined sea/island structure reducing portion.

Best Mode for Carrying Out the Invention

An embodiment of the present invention will be described in detail below.
A polymer for forming an extra fine filament yarn containing a deodorant functional agent according to the invention is not particularly limited, and may be any crystalline thermoplastic polymer having a fiber forming ability. Examples of the polymers include polyesters such as polyethylene terephthalate, polybutylene terephthalate, and polytrimethylene terephthalate, and polyamides such as nylon 6 and nylon 66. The polymer is preferably a polyethylene terephthalate, which is versatile and excellent in balance between costs and properties.
Extra fine single yarn fibers contained in the deodorant functional agent-containing extra fine multifilament of the invention have an average diameter of 200 to 2000 nm in a cross-section perpendicular to the axial direction. When the average diameter is less than 200 nm, the deodorant functional agent particles are aggregated, whereby it is difficult to form the filament yarn. On the other hand, when the average diameter is more than 2000 nm, the resultant multifilament does not have a soft texture, the exposed photocatalytic area is reduced in the case of using small deodorant functional agent particles, and the multifilament has a small specific surface area, whereby the photocatalytic reaction efficiency is deteriorated. The average diameter is preferably 300 to 1000 nm.
In the case of using a photocatalyst as the deodorant functional agent, the photocatalyst absorbs a light to exhibit the function. However, when the photocatalyst is kneaded into the filament, generally the fiber polymer per se inhibits the light absorption and the contact between the photocatalyst and a decomposition subject, thereby lowering the efficiency.
In addition, also when an adsorbent is kneaded into the filament as the deodorant functional agent, the fiber polymer per se inhibits the absorption function of the adsorbent, thereby lowering the efficiency.
Thus, the deodorant functional agent-containing extra fine multifilament of the invention contains at least the deodorant functional agent having a secondary particle diameter more than the average diameter of the extra fine single yarn fibers, and the deodorant functional agent is not completely covered with the fiber polymer. As a result, the deodorant efficiency is dramatically improved.
In general, it is difficult to produce a filament yarn, particularly an extra fine filament yarn, containing particles larger than the fiber diameter, because the spinnability is deteriorated by the particles in the production.
However, in the invention, it has found that the above extra fine multifilament can be produced by the steps of adding the deodorant functional agent to an island component of a sea-island composite filament yarn, and removing a sea component from the yarn to obtain the extra fine filament yarn containing the island component. Even when the island component has a brittle fiber structure (a structure containing the particles having a secondary particle diameter more than the extra fine single yarn fiber diameter, thus a low-strength structure), the sea-island composite filament yarn can have a strength sufficient for spin-drawing due to the sea component. Further, even after removing the sea component, the resultant extra fine filament yarn can have a sufficient strength due to the multifilament structure.
In the preparation of the sea-island composite filament yarn, it is preferred that a spun composite yarn is not winded up temporarily and is successively subjected to a drawing treatment in a spinning-direct drawing method. In the spinning-direct drawing method, the deodorant functional agent particles having the secondary particle diameter equal to or more than the extra fine single yarn fiber diameter cannot follow the stretch of the fiber polymer. Therefore, the area of the particles, not covered with the fiber polymer and exposed on the yarn surface, is increased. In view of improving the balance between the deodorant effect and fiber properties, 5 or more particles of the deodorant functional agent per 25 $\mu m^2$ are not completely covered with the fiber polymer and are partially exposed on the surface of the multifilament.

It should be noted that, when excessively many particles are exposed on the multifilament surface, the qualities (the strength, elongation, and fluff) of the multifilament are remarkably deteriorated disadvantageously. Thus, the number of the particles exposed on the multifilament surface is preferably 25 or less per 25 $\mu m^2$.

In this embodiment, the deodorant functional agent acts as a foreign substance in the fiber polymer. Each portion containing the deodorant functional agent has a low fiber polymer content, and thereby cannot follow the stretch of the fiber polymer. Thus, the portion is cracked, and the deodorant functional agent is partially separated from the fiber polymer therein to form an exposed area. In the exposed area, light incident and photocatalytic decomposition of a subject component are efficiently carried out, so that the deodorant function is dramatically improved.

The cross-sectional shape of the deodorant functional agent-containing extra fine filament yarn of the invention is not particularly limited, and may be a modified cross-sectional shape. Specific examples of the modified cross-sectional shapes include, but not limited thereto, T shapes, U shapes, V shapes, H shapes, Y shapes, W shapes, 3- to 14-leaf shapes, and polygonal shapes. The filament yarn may have a solid or hollow fiber structure.

In the invention, the photocatalyst used as the deodorant functional agent may be an oxidation photocatalyst, which generates active radicals under irradiation of a light such as an ultraviolet ray to oxidation-decompose various harmful substances and bad odor substances.

Thus, the photocatalyst preferably has an oxidation activity. Such a photocatalyst exhibits a deodorant function utilizing not only adsorbent but also catalytic decomposition, and thereby can maintain a deodorant or deodorization effect over a long period. Furthermore, the photocatalyst has a bactericidal effect, an antibacterial effect, etc. in addition to the effect of decomposing the harmful substances and bad odor substances.

The photocatalyst may be an inorganic or organic substance, and may be selected from various optical semiconductors. The photocatalyst is generally an inorganic optical semiconductor, and examples thereof include sulfide semiconductors (such as $CdS$, $ZnS$, $In_2S_3$, $PbS$, $Cu_2S$, $MoS_3$, $WS_2$, $Sb_3S_3$, $Bi_3S_3$, and $ZnCdS_2$), metal chalcogenides (such as $CdSe$, $In_2Se_3$, $WSe_3$, $HgSe$, $PbSe$, and $CdSe$), and oxide semiconductors (such as $TiO_2$, $ZnO$, $WO_3$, $CdO$, $In_2O_3$, $Ag_2O$, $MnO_2$, $Cu_2O$, $Fe_2O_3$, $V_2O_5$, and $SnO_2$). The examples further include semiconductors other than sulfide and oxide semiconductors, such as $GaAs$, $Si$, $Se$, $CdP_3$, and $Zn_2P_3$. The photocatalysts may be used singly or as a combination of two or more.

Among the above photocatalyst, preferred are sulfide semiconductors (such as $CdS$ and $ZnS$) and oxide semiconductors (such as $TiO_2$, $ZnO$, $SnO_2$, and $WO_3$), and particularly preferred are $TiO_2$ oxide semiconductors. The crystal structure of the optical semiconductor for the photocatalyst is not particularly limited. For example, the $TiO_2$ semiconductor may be of anatase type, brookite type, rutile type, amorphous type, etc. Particularly preferred $TiO_2$ semiconductors include anatase-type titanium oxides.

The photocatalyst may be used in the state of a sol, a gel, or a powder (particles). In the case of using the sol or gel of the photocatalyst, it is dried, solidified, and crushed into a desired particle diameter. The crushing may be carried out using a ball mill, a jet mill, etc., but the apparatus is not limited thereto. In the case of using the powder of the photocatalyst, the average secondary particle diameter of the photocatalyst is preferably 0.1 to 2 $\mu m$, more preferably 0.2 to 1.5 $\mu m$. When the particle diameter of the photocatalyst is more than 2 $\mu m$, for example, filter clogging or fluff yarn breakage is often caused in the melt spinning step, and yarn breakage is increased in the drawing step.

The amount of the photocatalyst used may be selected from a wide range depending on the fiber structure, as long as the catalytic activity is not deteriorated. The ratio of the photocatalyst to the entire filament yarn is, for example, 0.1% to 25% by mass, preferably 0.3% to 20% by mass, more preferably 0.5% to 10% by mass.

In the invention, the deodorant functional agent may comprise an adsorbent, a deodorant, an antibacterial agent, a bacteriostatic agent, or a combination thereof.

The adsorbent, deodorant, antibacterial agent, bacteriostatic agent cannot be clearly distinguished, and are not particularly limited. Examples of the agents include those containing, as main component, at least one selected from the group consisting of tetravalent metal phosphates, divalent metal hydroxides, silver oxides, zinc oxides, aluminum oxides, silicon oxides, zirconium oxides, silver ions, copper ions, and zinc ions. Specifically, the agent may be selected from MIZUKANITE available from Mizusawa Industrial Chemicals, Ltd., LIONITE available from Lion Corporation, hydrotalcite compounds available from Kyowa Chemical Industry Co., Ltd., KESMON series and NOVARON series available from Toagosei Co. , Ltd., KD-211GF available from Rasa Industries, Ltd., TZ-100 and SZ-100S available from Titan Kogyo, Ltd., and mixtures thereof, etc.

The average secondary particle diameter of the deodorant functional agent is preferably 0.1 to 2 $\mu m$, more preferably 0.2 to 1.5 $\mu m$. When the particle diameter is more than 2 $\mu m$, for example, filter clogging or fluff yarn breakage is often caused in the melt spinning step, and yarn breakage is increased in the drawing step.

The amount of the deodorant functional agent used may be selected from a wide range as long as the qualities (the strength, elongation, and fluff) of the filament yarn are not deteriorated. The ratio of the agent to the entire filament yarn is, for example, 0.1% to 25% by mass, preferably 0.3% to 20% by mass, more preferably 0.5% to 10% by mass.

The deodorant functional agent may be attached to the island component polymer as described above as follows: 1. the deodorant functional agent is added in or immediately after the polymerization of the island component polymer; 2. a masterbatch containing a base of the island component polymer and the deodorant functional agent is prepared and used; or 3. the deodorant functional agent is added in an optional step (such as a polymer pellet preparation step or a melt spinning step) before completion of the spinning; etc. The method using the masterbatch is preferred from the viewpoint of preventing side reactions due to the catalytic activity in the polymerization, etc.

In the deodorant functional agent-containing extra fine multifilament of the invention, a plurality of the deodorant functional agents may be used in combination to improve the catalytic function.

The sea-island composite filament yarn used in the invention may be prepared by using a known sea-island composite spinneret as shown in Figs. 1 and 2. The island component and the sea component are extruded in the melt states, and the resultant fibers are melt-spun at a rate of 500 to 3500 m/minute and then subjected to a drawing treatment and a heat treatment without temporal winding.

As the number of the islands is larger, the fibers of the island component are thinner after dissolution in the sea. The number of the islands is preferably 100 to 1, 000 per single yarn. When the number is less than 100, the resultant yarn has a low island ratio and cannot show properties specific to the extra fine filament yarn. On the other hand, when the number is more than 1,000, costs for producing the spinneret are increased, and its processing accuracy is often deteriorated. The number is further preferably 500 to 1,000.

The dissolution rate of the sea component is preferably 30 to 5,000 times, more preferably 100 to 4,000 times as high as that of the island component. When the dissolution rate ratio is less than 30 times, in a cross-section of the filament yarn, the separate island component is partially dissolved in a surface portion, and the sea component is often not dissolved in a center portion. As a result, the thickness of the island component is made uneven to deteriorate the quality. On the other hand, when the dissolution rate ratio is more than 5,000 times, it is difficult to form the filament yarn.

The sea component polymer for forming the sea-island composite filament yarn may be any fiber forming polymer such as polyamide, polystyrene, polyethylene, or polyester, as long as a difference between it and the island component in the solvent dissolution rate is 30 times or more. Particularly polyesters are preferred in view of controlling the solvent solubility. For example, the sea component polymer may be a polymer soluble in an alkaline aqueous solution of potassium hydroxide, sodium hydroxide, etc., which is optimally a polylactic acid, a polyethylene glycol-based polyester copolymer, or a polyester copolymer of 5-sodium sulfonate isophthalate. It is particularly preferred that the sea-island composite filament yarn is knitted or woven, and the sea component is dissolved and removed by a known alkali weight loss apparatus to obtain the extra fine yarn. It should be noted that nylon 6 is soluble in formic acid, and polystyrene is soluble in an organic solvent such as toluene.

The island component polymer may be any fiber forming polymer such as polyamide, polystyrene, polyethylene, or polyester, and is preferably a polyethylene terephthalate.

The shape of the deodorant functional agent-containing extra fine filament yarn of the invention in the length direction is not particularly limited. Thus, the filament yarn may be a yarn having a substantially constant diameter in the length direction, a thick and thin yarn having a diameter variation in the length direction, or another yarn. The photocatalyst-containing extra fine filament yarn may comprise a short or long fiber, and may be a spun yarn, a multifilament yarn, or a composite yarn of short and long fibers. The filament yarn of the invention may be subjected to an optional processing or treatment such as a false-twist processing, an air entanglement treatment (e.g. an interlacing processing), a crimping processing, an antishrink treatment, an anticrease treatment, a hydrophilization processing, a waterproofing processing, or an antidyeing processing, depending on the application or fiber type.

The deodorant functional agent-containing extra fine filament yarn of the invention may contain a common additive in addition to the deodorant functional agent depending on the fiber type. Examples of such additives include antioxidants, flame retardants, antistatics, colorants, lubricants, insect repellents, tick repellents, antifungals, ultraviolet absorbents, and flatting agents.

The deodorant functional agent-containing extra fine filament yarn of the invention can be used in various fibrous products. Examples of such products include yarns, clothes (such as woven fabrics, knitted fabrics, and nonwoven fabrics), pile clothes (such as woven pile fabrics and knitted pile fabrics), apparel clothes and other wears produced therefrom, interior products, bedclothes, and food packing materials. Specific examples thereof include apparel clothes and other wears such as underwears, sweaters, jackets, pajamas, summer kimonos (yukatas), white garments, slacks, socks, gloves, stockings, aprons, masks, towels, handkerchiefs, supporters, headhands, caps, shoe insoles, and padding clothes, and further include carpets, curtains, shop curtains (norens), wallpapers, sliding screen papers (shoji papers), sliding paper doors (fusumas), fiber blinds, artificial houseplants, fabrics for chairs, tableclothes, electric apparatus covers, straw mats (tatamis), fillings (e.g. cotton fillings) and side fabrics for Japanese beddings (futons), sheets, blankets, futon covers, pillows, pillow cases, bedcovers, fillings for beds, mats, hygiene materials, toilet seat covers, wiping clothes, and filters for air cleaners and air conditioners.

For example, under irradiation of a solar light, a fluorescent lamp, an ultraviolet ray lamp, etc., the deodorant functional agent-containing extra fine filament yarn of the invention and the fibrous product containing the filament yarn can decompose various odorous components and make the components odorless rapidly over a long period. The odorous components include basic odorous components (such as ammonia and amines), acidic odorous components (such as acetic acid), and neutral odorous components (such as formalin and acetaldehyde). Thus, the filament yarn can efficiently remove even an odor containing many odorous components, such as a cigarette odor, and thereby is useful for deodorizing rooms and cars. In addition, the filament yarn is useful also for deodorizing aldehydes such as formalin or acetaldehyde generated from furnitures and new building materials.

In the light irradiation, the light wavelength may be selected depending on the photocatalyst. The light wavelength is not limited as long as the photocatalyst can be activated, and the light generally contains an ultraviolet ray. The titanium oxide used as the photocatalyst can show a sufficiently effective catalytic activity even under a solar light or a fluorescent lamp light. The light irradiation is generally carried out in the presence of oxygen or an oxygen-containing matrix such as air.

Examples

The present invention will be described more specifically with reference to Examples and Comparative Examples below. It is to be understood that the invention is not limited to Examples, and various modifications may be made therein without departing from the scope of the invention. Characteristic values described in Examples were measured as follows.

(1) Deodorizing function

Deodorizing function was evaluated using a deodorization rate by the following method.

Using ammonia as an odorous component, 3 L of a gas having an initial odorous component concentration of 100 ppm was enclosed together with 1 g of a cylindrically knitted sample in a Tedlar bag. In the case of using a photocatalyst as a deodorant functional agent, the sample was irradiated with an ultraviolet ray lamp at 1.2 mW/cm$^2$·hr. After 24 hours, the residual odorous component concentration in the container was measured by a detecting tube. A blank examination was carried out in the same manner except for not using the sample. The deodorization rate was calculated using the following equation.

$$\texttt{Deodorization rate (\%) = } 100 \times (C_0 - C_1)/C_0$$

$C_0$: Concentration measured after 24 hours in blank examination
$C_1$: Concentration measured after 24 hours in Tedlar bag containing sample

(2) Number of exposed particles

A photograph of a side of drawn yarns arranged in parallel was taken at 5000 magnification using a scanning electron microscope. The number of particles exposed between cracked fiber surfaces per 25 $\mu m^2$ was counted, and the average value was calculated at n=10.

(3) Number of portions thicker than single yarn diameter.

In the same manner as the measurement of the exposed particle number, using the above photograph, the number of portions clearly thicker than the average single yarn diameter per 25 $\mu m^2$ was counted, and the average value was calculated at n=10.

(4) Average secondary particle diameter of deodorant functional agent

The average secondary particle diameter of the deodorant functional agent can be measured by various methods. For example, the particle diameter can be measured using a dynamic light scattering particle size distribution measuring apparatus. For example, MICROTRAC UPA (model 9340-UPA150) manufactured by Nikkiso Co., Ltd. may be used as the particle size distribution measuring apparatus.

[Example 1]

A polyethylene terephthalate having an intrinsic viscosity of 0.64 (at 35°C, in orthochlorophenol) was used as a base polymer, 10% by weight of a masterbatch was chip-blended with the base polymer, and the resultant was melted at 285°C by an extruder. The masterbatch was prepared using 10 parts by weight of a deodorant functional agent (a titanium oxide photocatalyst ST-01 available from Ishihara Sangyo Kaisha, Ltd.), which had an average secondary particle diameter of 1.2 μm measured by MICROTRACUPA (model 9340-UPA150) manufactured by Nikkiso Co., Ltd.

A modified polyethylene terephthalate copolymerized with 4 wt% of a polyethylene glycol (PEG) having an average molecular weight of 4000 and a melt viscosity of 1600 poise at 285°C and 8 mol% of 5-sodium sulfoisophthalate (SIP) as a sea component was melted by another extruder.

The photocatalyst-containing polyester was used as an island component, and each of the melted polymers were extruded at a sea/island component weight ratio of 30/70 from a spinneret having an island number of 836 at a spinning temperature of 285°C. The extruded polymers were pulled at a spinning speed of 1000 m/minute, and drawn at a preheating temperature of 90°C, a heat setting temperature of 140°C, and a draw ratio of 4.0 without temporal winding. The resultant was winded at 3950 m/minute to obtain a 56-dtex/10-fil drawn yarn.

A cylindrically knitted sample was prepared using the obtained drawn yarn, and its weight was reduced by 30% at 55°C by a 2.5% aqueous NaOH solution. As a result of observing a cross-section of the filament yarn, a uniform aggregate of extra fine fibers was formed, and the extra fine single yarn fibers had an average diameter of 690 nm. Furthermore, thick and thin portions containing the deodorant functional agent particles thicker than the fiber diameter were found on the side surface of the filament yarn. The deodorant functional agent particles thinner than the fiber diameter were directly observed in cracks on the yarn surface. As a result of evaluating the deodorizing function of the cylindrically knitted sample, the sample exhibited a deodorization rate of 100%. The results are shown in Table 1.

[Example 2]

Production and evaluation were carried out in the same manner as Example 1 except for changing the extrusion amount to prepare extra fine single yarn fibers having an average diameter of 385 nm. The results are shown in Table 1.

[Comparative Example 1]

Production and evaluation were carried out in the same manner as Example 1 except for not using the photocatalyst masterbatch. The results are shown in Table 1.

[Comparative Example 2]

Production and evaluation were carried out in the same manner as Example 1 except for using another spinneret in the spinning to prepare extra fine single yarn fibers having an average diameter of 2510 nm. The results are shown in Table 1.

[Comparative Example 3]

Production and evaluation were carried out in the same manner as Example 1 except for significantly reducing the extrusion amount to prepare extra fine single yarn fibers having an average diameter of 153 nm. However, the resultant single yarn fibers were extensively broken in the spinning and drawing, so that a sample usable for the deodorizing function evaluation could not be produced. The results are shown in Table 1.

[Example 3]

Production and evaluation were carried out in the same manner as Example 1 except that LIONITE PC available from Lion Corporation (average secondary particle diameter 3 μm) was used instead of the above photocatalyst and crushed into an average particle diameter of 1.9 μm using a jet mill manufactured by Seishin Enterprise Co. , Ltd. (model STJ-200). The results are shown in Table 1.

[Comparative Example 4]

Production and evaluation were carried out in the same manner as Example 3 except for not crushing the LIONITE PC available from Lion Corporation (average secondary particle diameter 3 μm). However, the resultant single yarn fibers were extensively broken in the spinning and drawing, so that a sample usable for the deodorizing function evaluation could not be produced. The results are shown in Table 1.

[Example 4]

Production and evaluation were carried out in the same manner as Example 1 except that an adsorbent MIZUKANITE HF available from Mizusawa Industrial Chemicals, Ltd. (average secondary particle diameter 2.7 μm) was

used instead of the above photocatalyst and crushed into an average particle diameter of 1.9 μm using a jet mill manufactured by Seishin Enterprise Co., Ltd. (model STJ-200). The results are shown in Table 1.

[Comparative Example 5]

Production and evaluation were carried out in the same manner as Example 4 except for not crushing the MIZUKANITE HF available from Mizusawa Industrial Chemicals, Ltd. (average secondary particle diameter 2.7 μm). However, the resultant single yarn fibers were extensively broken in the spinning and drawing, so that a sample usable for the deodorizing function evaluation could not be produced. The results are shown in Table 1.

[Example 5]

Production and evaluation were carried out in the same manner as Example 1 except for using a deodorant KESMON NS-10 available from Toagosei Co., Ltd. (average secondary particle diameter 0.9 μm) instead of the above photocatalyst. The results are shown in Table 1.

[Example 6]

Production and evaluation were carried out in the same manner as Example 1 except for using an antibacterial agent NOVARON AG300 available from Toagosei Co., Ltd. (average secondary particle diameter 0.9 μm) instead of the above photocatalyst. The results are shown in Table 1.

The samples of Examples 2 to 6, as well as the sample of Example 1, included in the scope of the invention were excellent in the deodorizing function. In contrast, the photocatalyst was not used in Comparative Example 1, and the numbers of the exposed photocatalyst particles and the portions thicker than the fiber diameter were significantly small in Comparative Example 2, whereby the samples of Comparative Examples 1 and 2 were remarkably poor in the deodorizing function. The results are shown in Table 1.

Table 1

| | Agent type | Agent manufacturer | Agent grade | Average secondary particle diameter of agent ($\mu$m) | Number of exposed particles (per 25 $\mu$m$^2$) | Number of portions thicker than single yarn diameter (per 25 $\mu$m$^2$) | Average single yarn diameter (nm) | Deodorization rate (%) | Note |
|---|---|---|---|---|---|---|---|---|---|
| Example 1 | Photo-catalyst | Ishihara Sangyo Kaisha, Ltd. | ST-01 | 1.2 | 12 | 12 | 690 | 100 | |
| Example 2 | Photo-catalyst | Ishihara Sangyo Kaisha, Ltd. | ST-01 | 1.2 | 20 | 23 | 385 | 100 | |
| Example 3 | Photo-catalyst | Lion Corporation | LIONITE PC | 1.9 | 23 | 15 | 720 | 100 | |
| Example 4 | Adsorbent | Mizusawa Industrial Chemicals, Ltd. | MIZUKANITE HF | 1.9 | 22 | 12 | 695 | 100 | |
| Example 5 | Deodorant | Toagosei Co., Ltd. | KESMON NS-10 | 0.9 | 19 | 25 | 715 | 100 | |
| Example 6 | Anti-bacterial agent | Toagosei Co., Ltd. | NOVARON AG300 | 0.9 | 20 | 23 | 720 | 100 | |
| Comparative Example 1 | - | - | - | - | 0 | 0 | 695 | 10 | |
| Comparative Example 2 | Photo-catalyst | Ishihara Sangyo Kaisha, Ltd. | ST-01 | 1.2 | 0.2 | 0.1 | 2510 | 20 | |
| Comparative Example 3 | Photo-catalyst | Ishihara Sangyo Kaisha, Ltd. | ST-01 | 1.2 | 5 | 8 | 153 | - | Sample not obtained |
| Comparative Example 4 | Photo-catalyst | Lion Corporation | LIONITE PC | 3.0 | 30 | 4 | 695 | - | Sample not obtained |

| | Agent type | Agent manufacturer | Agent grade | Average secondary particle diameter of agent ($\mu$m) | Number of exposed particles (per 25 $\mu$m$^2$) | Number of portions thicker than single yarn diameter (per 25 $\mu$m$^2$) | Average single yarn diameter (nm) | Deodori-zation rate (%) | Note |
|---|---|---|---|---|---|---|---|---|---|
| Comparative Example 5 | Adsorbent | Mizusawa Industrial Chemicals, Ltd. | MIZUKANITE HF | 2.7 | 29 | 5 | 700 | - | Sample not obtained |

**Claims**

**1.** A method for producing a deodorant functional agent-containing extra fine multifilament, comprising removing a sea component from a sea-island composite filament yarn including the sea component and island component to obtain an extra fine multifilament containing an island component, **characterized in that**

a) the island component comprises extra fine single yarn fibers having an average diameter of 200 to 2000 nm,
b) the island component comprises at least a deodorant functional agent having a secondary particle diameter equal to or more than the diameter of the extra fine single yarn fibers, and
c) the sea-island composite filament yarn is prepared by melt spinning and direct drawing without temporal winding.

**Patentansprüche**

**1.** Verfahren zur Herstellung eines extrafeinen Multifilaments mit einem Gehalt an Desodorierungsmittel, bei dem man aus einem zum Typ Inseln im See gehörenden Verbundfilamentgarn mit See- und Inselkomponente die Seekomponente entfernt und dabei ein extrafeines Multifilament mit der Inselkomponente erhält, **dadurch gekennzeichnet, dass**

a) die Inselkomponente extrafeine Einzelgarnfasern mit einem mittleren Durchmesser von 200 bis 2000 nm umfasst,
b) die Inselkomponente wenigstens ein Desodorierungsmittel mit einem sekundären Teilchendurchmesser größer gleich dem Durchmesser der extrafeinen Einzelgarnfasern umfasst und
c) die Herstellung des zum Typ Inseln im See gehörenden Verbundfilamentgarns durch Schmelzspinnen und Direktverstreckung ohne vorübergehendes Aufwickeln erfolgt.

**Revendications**

**1.** Procédé de production d'un multifilament extrafin contenant un agent désodorisant fonctionnel, comprenant le retrait d'un composant de type mer d'un fil continu composite de type mer-îlot comportant le composant de type mer et un composant de type îlot pour obtenir un multifilament extrafin contenant un composant de type îlot, **caractérisé en ce que**

a) le composant de type îlot comprend des fibres de fil simple extrafines ayant un diamètre moyen de 200 à 2000 nm,
b) le composant de type îlot comprend au moins un agent désodorisant fonctionnel ayant un diamètre de particules secondaires égal ou supérieur au diamètre des fibres de fil simple extrafines, et
c) le fil continu composite de type mer-îlot est préparé par filage à l'état fondu et étirage direct sans bobinage temporaire.

Fig. 1

Fig. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001254281 A
- JP 2005220471 A
- JP 2004169217 A
- JP 10204727 A
- US 2005136100 A
- WO 2007078203 A
- WO 2008000198 A
- JP 2001355119 A